# EUROPÄISCHE PATENTANMELDUNG

(11) **EP 3 025 759 A1**
(43) Veröffentlichungstag der Anmeldung: **01.06.2016**
(21) Anmeldenummer: 15190499.2
(22) Anmeldetag: 20.10.2015
(51) Int. Cl.: A61N 1/39, A61N 1/08, A61N 1/37

(54) **MRT-TAUGLICHES AKTIVES IMPLANTIERBARES GERÄT**

(30) Priorität: 27.11.2014 US 201462085261 P
(71) Anmelder: BIOTRONIK SE & Co. KG, 12359 Berlin (DE)
(72) Erfinder: Dörr, Thomas, 12437 Berlin (DE)
(74) Vertreter: Galander, Marcus

(57) **Zusammenfassung**

Die Erfindung betrifft ein MRT taugliches elektronisches implantierbares Gerät mit einer Steuereinheit (54, 420) und einer Speichereinheit (80), in der Programminformation zu der die Funktion der Steuereinheit (54, 420) steuernden Steuerprogrammen und/oder Steuerparameter, sowie wenigstens zeitweise jeweils aktuelle Zustandsparameter gespeichert sind, sowie mit einem MRT-Sensor (82), der mit der Steuereinheit (54, 420) verbunden und ausgebildet ist, auf eine Positionierung des Implantates/Patienten innerhalb oder in unmittelbarer Umgebung eines MRT-Gerätes anzusprechen und diese anzuzeigen. Außerdem weist das elektronische implantierbare Gerät (10) eine Statistikeinheit (440) auf, die mit der Steuereinheit (54, 420) verbunden oder Teil derselben ist und die ausgebildet ist, jeweils vor einem jeweiligen Ansprechen des MRT-Sensors (82) vorliegende aktuelle Zustandsparameter zu erfassen, wobei die Steuereinheit (54, 420) ausgebildet ist, bei Ansprechen des MRT-Sensors (82) mittels Auswertung der von der Statistikeinheit (440) erfassten Zustandsparameter ein durch diese Zustandsparameter indiziertes Steuerprogramm und/oder durch diese Zustandsparameter indizierte Steuerparameter auszuwählen und so-lange beizubehalten, solange der MRT-Sensor (82) eine Positionierung des elektronischen implantierbaren Gerätes (10) innerhalb oder in unmittelbarer Umgebung eines MRT-Gerätes anzeigt.

## Beschreibung

Die Erfindung betrifft ein MRT-taugliches elektronisches implantierbares Gerät, insbesondere ein implantierbares Herz-Therapie- und/oder Herz-Monitoring-Gerät, beispielsweise einen Herzschrittmacher oder Kardioverter/Defibrillator.

Implantierbare Herz-Therapie- und/oder Herz-Monitoring-Geräte, z. B. Herzstimulatoren in Form von Herzschrittmachern oder Cardiovertern/Defibrillatoren, sind grundsätzlich bekannt. Solche Herzstimulatoren sind in der Regel an Elektrodenleitungen angeschlossen, die in einer Kammer eines Herzens oder in deren unmittelbarer Nähe Stimulations- und optional zusätzliche Defibrillationselektroden besitzen. Über eine Stimulationselektrode - genauer: einen oder mehrere Stimulationselektrodenpole - kann ein Herzschrittmacher einen elektrischen Stimulationsimpuls an das Muskelgewebe einer Herzkammer abgeben, um so eine stimulierte Kontraktion der Herzkammer hervorzurufen, sofern der Stimulationsimpuls eine ausreichende Intensität besitzt und das Herzmuskelgewebe (Myokard) sich nicht gerade in einer refraktären Phase befindet. Eine derartig stimulierte Kontraktion einer Herzkammer wird im Rahmen dieser Beschreibung als stimuliertes Ereignis bezeichnet, ein Stimulationsimpuls, der eine ausreichende Intensität besitzt, eine stimulierte Kontraktion einer Herzkammer hervorzurufen, wird als "überschwellig" bezeichnet. Kommt es zu einer natürlichen Kontraktion der Herzkammer, wird dies im Rahmen dieser Beschreibung als Eigenaktion oder als natürliches oder intrinsisches Ereignis bezeichnet. Eine Kontraktion, beispielsweise des rechten Atriums eines Herzens, wird als atriales Ereignis bezeichnet, welches beispielsweise ein natürliches atriales Ereignis sein kann, oder - im Falle eines atrialen Herzschrittmachers - auch ein stimuliertes atriales Ereignis. Im gleichen Sinne können natürliche (intrinsische) und stimulierte linksventrikuläre und rechtsventrikuläre Ereignisse unterschieden werden.

Eine lokale Erregung des Myokards breitet sich vom Erregungsort ausgehend per Reizleitung im Myokard aus und führt zu einer Depolarisation der Muskelzellen und damit zu einer Kontraktion des Myokards. Nach kurzer Zeit kommt es zu einer Repolarisation der Muskelzellen und damit zu einer Entspannung des Myokards. Während der Phase der Depolarisation sind die Herzmuskelzellen für eine Erregung unempfindlich, d.h. refraktär. Diese Zeit wird als Refraktärzeit bezeichnet. Die mit der Depolarisation und Repolarisation einhergehenden elektrischen Potenziale können wahrgenommen und deren zeitlicher Verlauf - als Elektrokardiogramm bezeichnet - ausgewertet werden.

In einem Elektrokardiogramm sind mit einer Kontraktion des Ventrikels einhergehende, eine Depolarisation der Herzmuskelzellen widerspiegelnde Aktionspotenziale als sogenannte Q-Zacke zu erkennen, während sich die mit der Entspannung des Myokards einhergehende Repolarisierung der Herzmuskelzellen in einer sogenannten T-Welle widerspiegelt.

Beim gesunden Menschen wird der jeweilige Herzrhythmus durch den vom autonomen Nervensystem gesteuerte Sinusknoten bestimmt. Dieser erregt per Reizleitung das rechte Atrium eines menschlichen Herzens und weiter über den AV-Knoten den (rechten) Ventrikel des Herzens. Ein vom Sinusknoten ausgehender natürlicher Herzrhythmus wird daher auch als Sinusrhythmus bezeichnet und führt zu jeweils natürlichen Kontraktionen der jeweiligen Herzkammer, die als natürliche (intrinsische) Ereignisse erfasst werden können.

Das Erfassen solcher natürlicher (intrinsischer) Ereignisse erfolgt durch Ableiten der elektrischen Potenziale des Myokards der jeweiligen Herzkammer mit Hilfe von Wahrnehmungselektroden, die Teil einer entsprechenden Elektrodenleitung sind. Dabei können die Wahrnehmungselektrodenpole gleichzeitig die Stimulationselektrodenpole sein und abwechselnd als Stimulations- und als Wahrnehmungselektrodenpol verwendet werden. Typischerweise ist für das Sensing - d.h. die Wahrnehmung intrinsischer Ereignisse - ein Wahrnehmungselektrodenpolpaar vorgesehen, das von zwei benachbarten Elektrodenpolen, nämlich einer Spitzenelektrode (Tip-Elektrode) und einer Ring-Elektrode, gebildet ist, von denen die Spitzenelektrode auch als Stimulationselektrodenpol dient. Auf diese Weise erfolgt eine bipolare Ableitung eines intrakardialen Elektrokardiogramms (IEGM). Dabei erfolgt die Wahrnehmung intrinsischer Ereignisse und die Stimulation im Ventrikel mit Hilfe einer ventrikulären Elektrodenleitung und die Stimulation und die Wahrnehmung intrinsischer Ereignisse im Atrium (im rechten Atrium) mit einer atrialen Elektrodenleitung, die separat an den jeweiligen Herzstimulator angeschlossen sind. Zusätzlich kann auch eine linksventrikuläre Elektrodenleitung vorgesehen sein, die typischerweise über den Coronarsinus und eine von diesem abzweigende Lateralvene in die Nähe des linken Ventrikels ragt und dort eine kleinflächige Stimulations- und/oder Wahrnehmungselektrode aufweisen kann.

Um den unterschiedlichen Bedürfnissen verschiedener Patienten gerecht werden zu können, können implantierbare Herzstimulatoren in verschiedenen Betriebsmodi betrieben werden. Die verschiedenen Stimulations- und Wahrnehmungsmodi werden in der Regel einheitlich mit einem Drei-Buchstaben-Code bezeichnet, von denen der erste Buchstabe den Stimulationsort bezeichnet (V = Ventrikel, A = Atrium, D = Ventrikel und Atrium), der zweite Buchstabe den Ort der Wahrnehmung bezeichnet (V = Ventrikel, A = Atrium, D = Ventrikel und Atrium) und der dritte Buchstabe die Betriebsart (I = Inhibiert, T = Getriggert, D = sowohl Inhibiert als auch Getriggert). Insbesondere für Zwei-Kammer-Herzschrittmacher im DDD-Modus ist es auch bekannt, eine ventrikuläre Stimulation synchron zu einer möglichst natürlichen atrialen Herzrate vorzunehmen. Falls im Atrium keine gesunde, natürliche Herzrate wahrzunehmen ist, beispielsweise im Falle einer atrialen Tachykardie oder einer atrialen Fibrillation, weisen grundsätzlich atriumsynchrone Herzschrittmacher häufig ein Modeswitching von einer atriumsynchronen ventrikulären Stimulation zu einer atriumasynchronen Stimulation im VVI-Modus auf, falls eine wahrgenommene atriale Rate außerhalb zulässiger Grenzen liegt. Weiterhin ist es bekannt, ventrikuläre Tachykardien im Rahmen einer Kardioversionstherapie durch Stimulation mit einer Stimulationsrate, die über der Tachykardierate liegt, zu behandeln.

Die Stimulationsmodi können durch entsprechende Steuerprogramme, die z. B. erfasste Ereignisse verarbeiten oder ignorieren, oder durch Steuerparameter eingestellt werden. So kann beispielsweise durch einen Steuerparameter die Erfassung von Ereignissen im Atrium und/oder Ventrikel aktiviert oder deaktiviert werden.

In Bezug auf die hierin verwendeten Bezeichnungen sei darauf hingewiesen, dass im Rahmen dieses Textes mit den Begriffen Stimulations- oder Wahrnehmungselektrode ein jeweiliger Elektrodenpol an einer Elektrodenleitung gemeint ist, also derjenige Teil einer Elektrodenleitung, über den Stimulationsimpulse abgegeben oder elektrische Potenziale aufgenommen werden. Es sei auch darauf hingewiesen, dass es auch üblich ist, mit "Stimulationselektrode" eine der Stimulation dienende Elektrodenleitung zu bezeichnen.

Die Wahrnehmungselektrodenpole sind im Betrieb des Herzstimulators mit entsprechenden Wahrnehmungseinheiten verbunden, die ausgebildet sind, ein jeweiliges über einen Wahrnehmungselektrodenpol (bzw. ein Wahrnehmungselektrodenpolpaar) aufgenommenes Elektrokardiogramm auszuwerten und insbesondere intrinsische atriale bzw. ventrikuläre Ereignisse zu detektieren, d.h. natürliche atriale oder ventrikuläre Kontraktionen. Dies geschieht beispielsweise durch Schwellwertvergleich, d.h. ein intrinsisches Ereignis wird detektiert, wenn ein jeweiliges intrakardiales Elektrokardiogramm einen geeignet vorgegebenen Schwellwert überschreitet.

Aus der Frequenz, mit der atriale bzw. ventrikuläre Ereignisse aufeinander folgen, kann die jeweilige intrinsische atriale Herzrate (atriale Frequenz) bzw. ventrikuläre Herzrate (Ventrikelfrequenz) abgeleitet und somit beispielsweise Tachykardien detektiert werden.

Das Erfassen natürlicher Ereignisse dient bei bekannten Demand-Schrittmachern außerdem der Unterdrückung (Inhibierung) der Abgabe von Stimulationsimpulsen an eine entsprechende Herzkammer, falls das natürliche Ereignis in einem Zeitfenster vor der geplanten Abgabe eines Stimulationsimpulses an diese Herzkammer erfasst wird. Bei ratenadaptiven Herzschrittmachern wird der Zeitpunkt der Abgabe eines jeweiligen Stimulationsimpulses in Abhängigkeit einer jeweiligen Stimulationsrate geplant, die dem physiologischen Bedarf eines Patienten entsprechen soll, also bei größerer Anstrengung beispielsweise höher ist. Hierzu kann ein Herzstimulator mit einem oder mehreren Aktivitätssensoren ausgestattet sein, der beispielsweise ein CLS-Sensor (CLS: Closed Loop Stimulation) sein kann.

Problematisch ist, dass solche implantierbaren elektrischen medizinischen Geräte wie Herzstimulatoren durch starke elektromagnetische Felder oder Magnetfelder, wie sie zum

Beispiel in einem Magnetresonanztomografen (MRT) auftreten, in ihrer Funktion stark beeinträchtigt werden können. Daher sind viele Träger von aktiven implantierbaren medizinischen Geräten (im Folgenden auch Implantat oder IMD genannt) für MRT-Untersuchungen kontraindiziert, obwohl MRT-Untersuchungen einen immer höheren Stellenwert in der diagnostischen Medizin erhalten.

Um Trägern von aktiven implantierbaren medizinischen Geräten dennoch MRT-Untersuchungen zu ermöglichen, sind verschiedene Ansätze, die sich entweder auf die Durchführung der MRT-Untersuchung oder auf das implantierbare medizinische Gerät beziehen, bekannt.

Unter anderem sind Technologien zur Erkennung von Magnetfeldern bekannt, die auf herkömmlichen Verfahren zur Magnetfelddetektion beruhen. So beschreibt die US 2008/0154342 ein Verfahren unter Ausnutzung eines GMR-Sensors (Giant Magnetic Resistance), um problematische Magnetfelder von MRT-Geräten zu erkennen.

Auch ist es bekannt, dass beispielsweise ein Implantierbarer Cardioverter/Defibrillator (ICD) vor einer MRT-Untersuchung durch einen Kardiologen in einen Betriebsmodus versetzt wird, der durch die im MRT herrschenden Magnetfelder nicht beeinträchtigt wird. Nach der MRT-Untersuchung durch einen Radiologen muss ein Kardiologe den ICD wieder in einen Betriebsmodus versetzen, der den Bedürfnissen des Patienten entspricht.

Hiervon ausgehend liegt der Erfindung der Wunsch zugrunde, die Tauglichkeit eines aktiven implantierbaren medizinischen Gerätes auch unter dem Einfluss von Magnetfeldern, wie sie für einen Magnetresonanztomographen (MRT) typisch sind, möglichst zu verbessern.

Hierzu wird erfindungsgemäß ein MRT-taugliches elektronisches implantierbares Gerät vorgeschlagen, das eine Steuereinheit und eine Speichereinheit aufweist. In der Speichereinheit ist Programminformation zu Steuerprogrammen und/oder Steuerparametern gespeichert, die die Funktion der Steuereinheit steuern. Außerdem sind in der Steuereinheit wenigstens zeitweise jeweils aktuelle Zustandsparameter gespeichert. Das elektronische implantierbare Gerät besitzt außerdem einen MRT-Sensor, der mit der Steuereinheit verbunden und ausgebildet ist, auf eine Positionierung des Implantates (oder eines Patienten mit dem Implantat) innerhalb oder in unmittelbarer Umgebung eines MRT-Gerätes anzusprechen und diese anzuzeigen. Erfindungsgemäß weist das elektronische implantierbare Gerät eine Statistikeinheit auf, die mit der Steuereinheit verbunden oder Teil derselben ist und die ausgebildet ist, jeweils vor einem jeweiligen Ansprechen des MRT-Sensors vorliegende aktuelle Zustandsparameter zu erfassen. Die Steuereinheit ist ausgebildet, bei Ansprechen des MRT-Sensors mittels Auswertung der von der Statistikeinheit erfassten Zustandsparameter ein durch diese Zustandsparameter indiziertes Steuerprogramm und/oder durch diese Zustandsparameter indizierte Steuerparameter auszuwählen und solange beizubehalten, solange der MRT-Sensor eine Positionierung des elektronischen implantierbaren Gerätes innerhalb oder in unmittelbarer Umgebung eines MRT-Gerätes anzeigt.

Zustandsparameter sind Parameter die entweder den Betriebszustand des implantierbaren elektronischen Gerätes oder einen Zustand eines Patienten oder beides beschreiben, zumal der Betriebszustand des implantierbaren elektronischen Gerätes typischerweise von dem jeweiligen aktuellen Zustand des jeweiligen Patienten abhängt. So hängt eine Stimulationsrate als ein Zustandsparameter des Gerätes z. B. von einem von dem Gerät erfassten Eigenrhythmus des Patienten ab. Der Eigenrhythmus ist der Rhythmus natürlicher Kontraktionen (Eigenaktionen, intrinsischer Ereignisse) einer Herzkammer.

Steuerprogramme oder Steuerparameter bestimmen die Funktionsweise, insbesondere den jeweiligen Betriebsmodus des implantierbaren elektronischen Gerätes, der typischerweise von der Steuereinheit gesteuert wird. In diesem Sinne beeinflussen in der Speichereinheit gespeicherte Steuerprogramme oder Steuerparameter die Arbeitsweise der Steuereinheit. Im vorliegenden Fall steuert die Steuereinheit nicht nur den Betrieb des implantierbaren elektronischen Gerätes hinsichtlich beispielsweise einer Therapieabgabe wie der Herzstimulation, sondern die Steuereinheit wählt auch ein jeweils anzuwendendes Steuerprogramm oder anzuwendende Steuerparameter aus, die ihrerseits dann wieder z. B. die Therapieabgabe definieren. Auch die Auswahl eines jeweils anzuwendenden Steuerprogramms oder anzuwendender Steuerparameter durch die Steuereinheit kann programmierbar sein, z. B. durch Speichern von von der Steuereinheit bei der Auswahl anzuwendender Auswahlkriterien in der Speichereinheit. Im Falle eines elektronischen implantierbaren Gerätes in Form eines Herzschrittmachers legen die Steuerprogramme oder Steuerparameter beispielsweise den Wahrnehmungs- und/oder Stimulationsmodus wie beispielsweise DDD oder V00 oder A00 fest.

Die Erfindung ermöglicht den sicheren Betrieb eines elektronischen Implantates in einem MRT, ohne dass unmittelbar vor der Untersuchung eine Umprogrammierung bzw. Anpassung des MRT-Programmes stattfinden muss.

Die Erfindung schließt die Erkenntnis ein, dass die bisher bekannten Lösungen und auch eine denkbare automatische MRT-Umprogrammierung den Nachteil haben, dass der Arzt, der das elektronische Implantat nachsorgt, ein für die MRT-Untersuchung geeignetes patientenindividuelles Programm im Implantat für die MRT-Umschaltung hinterlegen muss. Diese patientenindividuelle Einstellung kann sich aber im Laufe der Zeit verändern, d.h. der Arzt kann heute nicht sicher festlegen, welche Programmierung für einen Patienten in einigen Monaten oder Jahren während einer MRT-Untersuchung als sicher einzustufen ist.

In einer bevorzugten Ausführungsform ist das elektronische implantierbare Gerät ein implantierbarer Pulsgenerator (IPG) und/oder ein implantierbarer Cardioverter/Defibrillator (ICD) und/oder ein Herzstimulator für eine kardiale Resynchronisationstherapie (CRT) oder ein Neurostimulator. Besonders bevorzugt ist das elektronische implantierbare Gerät ein Herz-Therapie- und/oder Herz-Monitoring-Gerät, insbesondere ein implantierbarer, beispielsweise biventrikulärer Herzschrittmacher und/oder Kardioverter/Defibrillator. Mit einem biventrikulären Herzschrittmacher kann insbesondere eine kardiale Resynchronisations-Therapie (CRT) durchgeführt werden.

Vorzugsweise enthält die Speichereinheit ein von der Steuereinheit wählbares Steuerprogramm, das so konfiguriert ist, dass dieses Steuerprogramm ein Auslösen von Stimulationsimpulsen unterbindet. Die Speichereinheit beinhaltet somit mindestens ein Programm, das einen Betriebsmodus bewirkt, der ohne Stimulation ist.

Falls das implantierbare elektronische Gerät ein Herzschrittmacher ist, kann die Speichereinheit alternativ auch ein von der Steuereinheit wählbares Steuerprogramm oder wählbare Steuerparameter enthalten, das so konfiguriert ist bzw. die so konfiguriert sind, dass es die Steuereinheit veranlasst, eine asynchrone Stimulation in einem asynchronen Stimulationsmodus wie V00 oder D00 zu bewirken. In diesem Falle ist das elektronische programmierbare Gerät ein Herzstimulator wie beispielsweise ein Herzschrittmacher oder ICD. Gemäß dem weiter vorne beschriebenen Drei-Buchstaben-Code kommen diese Stimulationsmodi ohne Wahrnehmung intrinsischer Ereignisse aus, sodass auch keine Inhibierung oder Triggerung von Stimulationsimpulsen durch erfasste intrinsische Ereignisse erfolgt. Derartige Stimulationsmodi tragen dem Umstand Rechnung, dass ein Herzstimulator infolge wechselnder magnetischer oder elektromagnetischer Felder durch diese Felder induzierte Signale fälschlicherweise als intrinsische Ereignisse erfassen könnte. In einem reinen Stimulationsmodus, der ohne das Erfassen intrinsischer Ereignisse auskommt, besteht dieses Problem nicht.

Falls das implantierbare elektronische Gerät ein Herzschrittmacher ist, ist es weiter bevorzugt, wenn in der Speichereinheit ein Steuerprogramm abgelegt ist, das von der Steuereinheit gewählt werden kann und das so konfiguriert ist, dass es eine asynchrone Stimulation in einem asynchronen Stimulationsmodus wie VOO oder DOO bewirkt.

Weiterhin ist es im Falle eines elektronischen implantierbaren Gerätes in Form eines Herzstimulators bevorzugt, wenn die Statistikeinheit einen Stimulationstrendspeicher umfasst oder mit einem solchen verbunden ist, wobei die Statistikeinheit oder die Steuereinheit ausgebildet ist, ein nach Ansprechen des MRT-Sensors anzuwendendes Steuerprogramm oder nach Ansprechen des MRT-Sensors anzuwendende Steuerparameter unter Berücksichtigung von mehreren in dem Stimulationstrendspeicher in einem Zeitraum vor einem jeweiligen Ansprechen des MRT-Sensors abgelegten Zustandsparameter auszuwählen. Hierbei ist es insbesondere bevorzugt, wenn der Stimulationstrendspeicher Zustandsparameter enthält, die beispielsweise die Abgabe von Stimulationsimpulsen und/oder die Frequenz der Abgabe von Stimulationsimpulsen widerspiegeln.

Alternativ oder zusätzlich kann die Statistikeinheit und/oder die Steuereinheit ausgebildet sein, ein nach Ansprechen des MRT-Sensors anzuwendendes Steuerprogramm oder nach Ansprechen des MRT-Sensors anzuwendende Steuerparameter unter Berücksichtigung eines von dem Herzstimulator in einem Zeitraum von einem jeweiligen Ansprechen des MRT-Sensors erfassten Eigenrhythmus auszuwählen. In diesem Falle sind die die Auswahl des Steuerprogramms oder der Steuerparameter beeinflussenden Zustandsparameter solche Zustandsparameter, die den Zustand eines Patienten beschreiben, so wie er von dem elektronischen implantierbaren Gerät erfasst wird.

Alternativ oder zusätzlich können die Statistikeinheit und/oder die Steuereinheit auch ausgebildet sein, ein nach Ansprechen des MRT-Sensors anzuwendendes Steuerprogramm oder nach Ansprechen des MRT-Sensors anzuwendende Steuerparameter unter Berücksichtigung von von dem Herzstimulator in einem Zeitraum vor einem jeweiligen Ansprechen des MRT-Sensors erfassten Extrasystolen auszuwählen. Insbesondere können die Statistikeinheit und/oder die Steuereinheit ausgebildet sein, ein jeweiliges Steuerprogramm oder jeweilige Steuerparameter in Abhängigkeit eines erfassten Extrasystolentrends auszuwählen.

Unabhängig davon, welcher Art das elektronische implantierbare Gerät ist, kann die Speichereinheit in einer bevorzugten Ausführungsvariante ein Auswahlprogramm enthalten, dass die Auswahl eines nach Ansprechen des MRT-Sensors anzuwendenden Steuerprogramms oder nach Ansprechen des MRT-Sensors anzuwendender Steuerparameter durch die Statistikeinheit und/oder die Steuereinheit steuert. Damit sind auch die Kriterien zur Auswahl des Steuerprogramms oder der Steuerparameter die von der Statistikeinheit oder der Steuereinheit angewandt werden, programmierbar. Insbesondere kann die Speichereinheit neben den jeweiligen Steuerprogrammen und/oder Steuerparametern jeweils diesen Steuerprogrammen und/oder Steuerparametern zugeordnete Auswahlkriterien enthalten, die die Funktionsweise der Steuereinheit oder der Statistikeinheit beim Auswählen des jeweiligen Steuerprogramms oder der jeweiligen Steuerparameter bestimmen.

Jeweils auszuwählende Steuerprogramme oder Steuerparameter sind vorzugsweise grundsätzlich so gewählt, dass sie Einstellungen bewirken, die einen grundsätzlich sicheren Betrieb des implantierbaren elektronischen Gerätes in einem Magnetresonanztomographen gewährleisten, beispielsweise das Deaktivieren der Schockabgabe in einem ICD.

Vorzugsweise ist das implantierbare elektronische Gerät ausgebildet, zyklisch Testalgorithmen auszuführen, um Auswahlkriterien für die Auswahl eines nach Ansprechen des MRT-Sensors anzuwendenden Steuerprogramms oder nach Ansprechen des MRT-Sensors anzuwendender Steuerparameter regelmäßig automatisch zu aktualisieren.

Weiterhin kann das implantierbare elektronische Gerät in einer bevorzugten Ausführungsvariante derart ausgebildet sein, dass es einen oder mehrere Steuerparameter oder ein oder mehrere Steuerprogramme in Abhängigkeit von erfassten Zustandsparametern modifiziert. Beispielsweise kann eine nach Ansprechen des MRT-Sensors anzuwendende Stimulationsrate (vorgegeben durch entsprechende Steuerparameter) von dem implantierbaren elektronischen Gerät so eingestellt oder modifiziert werden, dass diese von gemessenen kürzesten Herzzyklen (der Dauer zwischen zwei intrinsischen Ereignissen) und der Häufigkeit von Extrasystolen abhängt. Die Stimulationsrate ist dann ein Steuerparameter, der von dem implantierbaren elektronischen Gerät in Abhängigkeit erfasster Zustandsparameter (kürzeste Herzzyklen, Häufigkeit von Extrasystolen) jeweils angepasst und somit modifiziert wird.

Vorzugsweise ist der MRT-Sensor ein Magnetfeldsensor und/oder ein Gradientenfeldsensor und/oder ein Hochfrequenz-Feldsensor und/oder ein Lagesensor und/oder ein (Lorenz-)Vibrationssensor und/oder ein Sensor, der charakteristische Spannungsverläufe beispielsweise in den Elektrodenleitungen überwacht.

Die Erfindung soll nun anhand von Ausführungsbeispielen mit Bezug auf die Figuren näher erläutert werden. Die Figuren zeigen in
- Figur 1:: Ein System mit einem Herz-Therapie- und/oder Herz-Monitoring-Gerät als implantierbarem elektronischen Gerät in Form eines Dreikammer-ICD-Systems;
- Figur 2:: die Hauptbestandteile des Herz-Therapie- und/oder Herz-Monitoring-Gerätes aus Figur 1;
- Figur 3:: einen typischen Ablauf einer MRT-Untersuchung eines ICD-Patienten ohne erfindungsgemäßes Gerät; und
- Figur 4:: ein weiteres Blockschaltbild eines elektronischen implantierbaren medizinischen Gerätes.

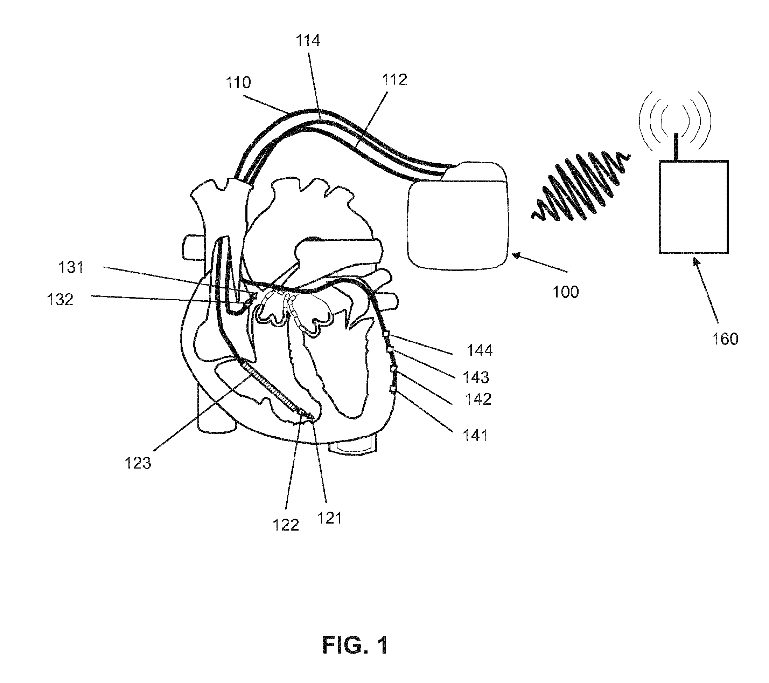

In Figur 1 ist als Beispiel für ein aktives implantierbares medizinisches Gerät ein Herz-Therapie- und/oder Herz-Monitoring-Gerät in Form eines Dreikammer-ICD-Systems dargestellt. Dieses umfasst einen Generator 100 (als das Herz-Therapie- und/oder Herz-Monitoring Gerät), der mit mehreren implantierbaren Elektrodenleitungen 110, 112 und 114 verbunden ist. Zur rechtsventrikulären Wahrnehmung und Stimulation ist eine rechtsventrikuläre (RV-) Elektrodenleitung 110 vorgesehen, die eine rechtsventrikuläre (RV-) Tip-Elektrode 121 und eine rechtsventrikuläre (RV-) Ring-Elektrode 122 am distalen Ende umfasst. Über die RV-Tip-Elektrode 121 können im Betrieb bei Bedarf rechtsventrikuläre Stimulationsimpulse für eine biventrikuläre CRT-Stimulation abgegeben werden. Zur Defibrillationsschockabgabe sind an dieser Elektrodenleitung 110 eine distale Schockwendel 123 und optional auch eine proximale Schockwendel (nicht dargestellt) angebracht. Die Gegenelektrode bildet hier das Generatorgehäuse des Generators 100.

Eine rechtsatriale Elektrodenleitung 112 weist an ihrem distalen Ende einen bipolaren Wahrnehmungs- und Stimulationspol mit einer rechtsatrialen Tip-Elektrode 131 und einer rechtsatrialen Ring-Elektrode 132 auf und dient der Wahrnehmung des atrialen Rhythmus und bei Bedarf der atrialen Stimulation.

Außerdem umfasst das System auch eine linksventrikuläre CS-Elektrodenleitung zur Abgabe von linksventrikulären Stimulationsimpulsen zur CRT über einen oder mehrere von insgesamt vier linksventrikulären (CS-) Stimulationselektrodenpolen 141, 142, 143 und 144.

Für eine Kommunikation mit externen Programmier- und Steuer- und Datenübertragungsgeräten 160 ist in dem Generator 100 eine drahtlose, bidirektionale Telemetrieeinheit vorgesehen.

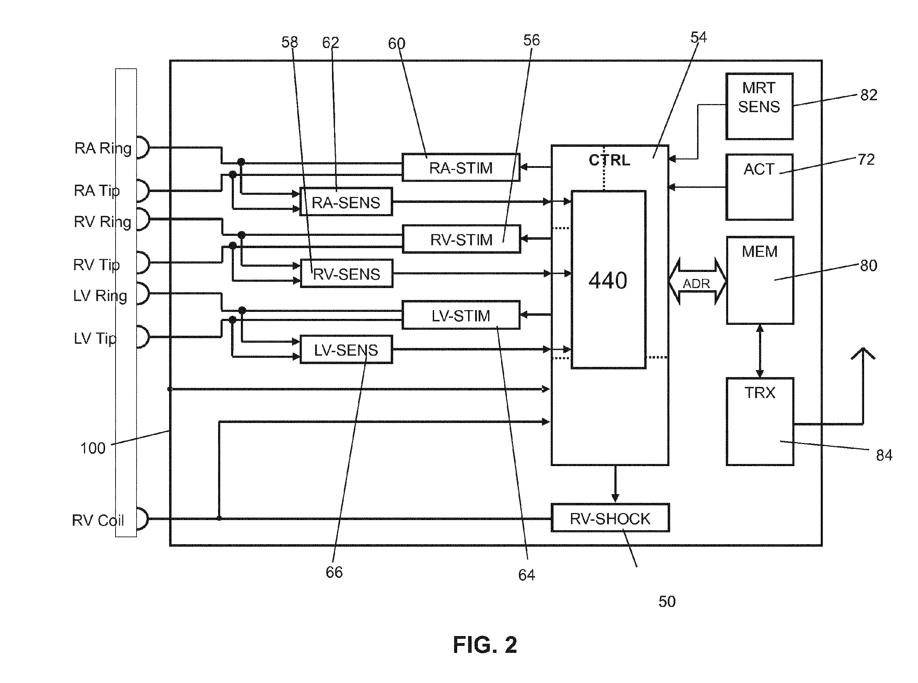

Figur 2 zeigt die Hauptbestandteile des Herzstimulators 100 aus Figur 1. Auf der linken Seite sind die elektrischen Anschlüsse für die verschiedenen Elektroden 121, 122, 131 und 132 dargestellt. Die Schockelektroden 123 sind mit einem rechtsventrikulären Schockimpulsgenerator 50 verbunden. Der Schockgenerator 50 ist mit einer Stimulationssteuereinheit 54 verbunden, die den Schockimpulsgenerator 50 bei Bedarf zur Erzeugung und Abgabe eines Defibrillationsschocks ansteuert.

Der Anschluss für die rechtsventrikuläre Tip-Elektrode 121 (RV Tip) sowie der Anschluss für die rechtsventrikuläre Ring-Elektrode 122 (RV Ring) sind jeweils sowohl mit einer rechtsventrikulären Stimulationseinheit 56 als auch mit einer rechtsventrikulären Wahrnehmungseinheit 58 verbunden. Sowohl die rechtsventrikuläre Stimulationseinheit 56 als auch die rechtsventrikuläre Wahrnehmungseinheit 58 sind jeweils mit der Stimulationssteuereinheit 54 verbunden.

Die rechtsventrikuläre Stimulationseinheit 56 ist dazu ausgebildet, auf ein Ansteuersignal der Stimulationssteuereinheit 54 hin einen rechtsventrikulären Stimulationsimpuls zu erzeugen und im Anschluss über die rechtsventrikuläre Ring-Elektrode RV Ring und die rechts-ventrikuläre Spitzenelektrode RV Tip abzugeben. Alternativ ist es auch möglich, dass das Gehäuse des Generators 100 eine neutrale Elektrode bildet und die rechtsventrikuläre Stimulationseinheit 56 mit dem Anschluss für die rechtsventrikuläre Ring-Elektrode RV Ring und dem Gehäuse als andere Elektrode zur Abgabe eines Stimulationsimpulses verbunden ist. Ein rechtsventrikulärer Stimulationsimpuls unterscheidet sich von einem Defibrillationsschock dadurch, dass der Stimulationsimpuls eine wesentlich geringere Impulsstärke besitzt, so dass er nicht wie ein Defibrillationsschock auf einen Schlag das vollständige Herzgewebe (Myokard) einer Herzkammer erregt, sondern nur die Herzmuskelzellen in unmittelbarer Umgebung der rechtsventrikulären Spitzenelektrode RV Tip 121. Diese Erregung breitet sich dann durch natürliche Reizleitung über den gesamten rechten Ventrikel weiter aus und sorgt so für eine stimulierte Kontraktion des rechten Ventrikels.

Die rechtsventrikuläre Wahrnehmungseinheit 58 ist dazu ausgebildet, an dem Anschluss für die rechtsventrikuläre Ring-Elektrode RV Ring 122 und die rechtsventrikuläre Spitzenelektrode RV Tip 121 anliegende elektrische Potenziale zunächst durch einen Eingangsverstärker zu verstärken und zu filtern. Weiterhin ist die rechtsventrikuläre Wahrnehmungseinheit 58 ausgebildet, den Verlauf der an ihren Eingängen anliegenden elektrischen Signale derart auszuwerten, dass die rechtsventrikuläre Wahrnehmungseinheit 58 selbsttätig eine natürliche (intrinsische), d.h. selbsttätige, Kontraktion des rechten Ventrikels detektiert. Dies kann beispielsweise dadurch geschehen, dass der Verlauf des an den Eingängen der rechtsventrikulären Wahrnehmungseinheit 58 anliegenden Signals mit einem Schwellenwert verglichen wird. Typischerweise ist die größte Amplitude des Signals in Form der sogenannten R-Zacke kennzeichnend für eine natürliche Kontraktion des rechten Ventrikels, die durch Schwellenwertvergleich detektiert werden kann. Die rechtsventrikuläre Wahrnehmungseinheit 58 gibt daraufhin ein entsprechendes, eine natürliche Kontraktion des rechten Ventrikels anzeigendes Ausgangssignal an die Stimulationssteuereinheit 54 aus. Der Zeitpunkt, an dem der Schwellenwert überschritten wird, ist der Erfassungszeitpunkt für das jeweilige Ereignis.

In gleicher Art und Weise können auch für die Schockelektroden eine oder mehrere (in Figur 2 nicht dargestellte) Wahrnehmungseinheiten vorgesehen sein. Diese Wahrnehmungseinheit oder Wahrnehmungseinheiten sind bevorzugt ausgebildet, Signale zwischen den Schockelektroden, zwischen der Schockelektrode 123 und dem Gehäuse des Generators 100 oder zwischen der anderen Schockelektrode und dem Gehäuse des Generators 100 zu erfassen.

In analoger Weise sind der Anschluss für die rechtsatriale Spitzenelektrode 131 (RA Tip) und der Anschluss für die rechtsatriale Ring-Elektrode 132 (RA Ring) sowohl mit einer rechtsatrialen Stimulationseinheit 60 als auch mit einer rechtsatrialen Wahrnehmungseinheit 62 verbunden, die jeweils ihrerseits wiederum mit der Stimulationssteuereinheit 54 verbunden sind. Die rechtsatriale Stimulationseinheit 60 ist dazu ausgebildet, Stimulationsimpulse zu erzeugen, deren Stärke ausreicht, das rechtsatriale Myokard zu erregen. Die rechtsatrialen Stimulationsimpulse können dabei eine andere Impulsstärke besitzen als die rechts-ventrikulären Stimulationsimpulse. Die rechtsatriale Wahrnehmungseinheit 62 ist ausgebildet, aus dem Verlauf des an ihren Eingängen anliegenden Differenzsignals eine sogenannte P-Welle zu detektieren, die eine natürliche (intrinsische) Kontraktion des rechten Atriums kennzeichnet. Detektiert die rechtsatriale Wahrnehmungseinheit 62 eine entsprechende P-Welle, erzeugt sie ein Ausgangssignal und gibt dieses an die Stimulationssteuereinheit 54 weiter, welches eine natürliche Kontraktion des rechten Atriums kennzeichnet.

In gleicher Weise sind auch der Anschluss für die linksventrikuläre Spitzenelektrode 141 (LV Tip) und die Anschlüsse für die linksventrikulären Ring-Elektroden 142, 143 und 144 (aus Vereinfachungsgründen ist nur ein Anschluss LV Ring dargestellt) mit einer linksventrikulären Stimulationseinheit 64 und einer linksventrikulären Wahrnehmungseinheit 66 verbunden. Die linksventrikuläre Stimulationseinheit 64 und die linksventrikuläre Wahrnehmungseinheit 66 sind ebenso mit der Stimulationssteuereinheit 54 verbunden. Beide funktionieren ähnlich wie die bereits beschriebenen Stimulationseinheiten 56 und 60 und Wahrnehmungseinheiten 58 und 62.

Als weiterer Bestandteil des Herzstimulators 10 ist ein Beschleunigungssensor 72 mit der Stimulationssteuereinheit 54 verbunden und in das Gehäuse 42 des Herzstimulators 10 integriert. Der Beschleunigungssensor 72 ist dazu ausgebildet, ein von der körperlichen Aktivität eines Patienten abhängiges Bewegungssignal zu erfassen und ein entsprechendes, die körperliche Aktivität des Patienten anzeigendes erstes Akzelerometer-Ausgangssignal an die Stimulationssteuereinheit 54 auszugeben. Dies erlaubt es, dass die Stimulationssteuereinheit 54 das Timing der Stimulationsimpulse an den Bedarf des Patienten (hämodynamischen Bedarf) anpasst. Das Akzelerometer-Ausgangssignal kann auch zum Bestimmen von Ruhephasen genutzt werden, in denen bevorzugt eine Dislokationsdetektion stattfinden kann.

Weiterhin umfasst der Generator 100 eine Speichereinheit 80, die mit der Stimulationssteuereinheit 54 verbunden ist und es erlaubt, von der Stimulationssteuereinheit 54 erzeugte oder ausgewertete Signale zu speichern. Andererseits erlaubt es die Speichereinheit 80, Steuerprogramme für die Stimulationssteuereinheit 54 in veränderbarer Form zu speichern.

Des Weiteren ist die Stimulationssteuereinheit 54 mit einem MRT-Sensor 82 verbunden. Der MRT-Sensor kann beispielsweise ein Magnetfeldsensor und/oder ein Gradientenfeldsensor und/oder ein Hochfrequenz-Feldsensor und/oder ein Lagesensor und/oder ein (Lorentz-)Vibrationssensor und/oder ein Sensor sein, der charakteristische Spannungsverläufe überwacht.

Die Speichereinheit 80 ist mit einer Telemetrieeinheit 84 verbunden, die es erlaubt, in der Speichereinheit 80 abgelegte Daten drahtlos an das externe Gerät 160 zu übertragen oder Programmierbefehle seitens des externen Geräts 160 zu dem Herzstimulator 10 zu übertragen und in der Speichereinheit 80 zu speichern.

Das erfindungsgemäße Gerät ist ein elektronisches Implantat mit einer automatischen MRT-Erkennung und Umschaltung in einen sicheren Zustand während einer MRT-Untersuchung, wobei in diesem Implantat mindestens zwei patientenindividuelle MRT-Programmeinstellungen hinterlegt sind, die in Abhängigkeit eines vom Implantat vor der MRT-Untersuchung erfassten Patientenstatus automatisch ausgewählt werden.

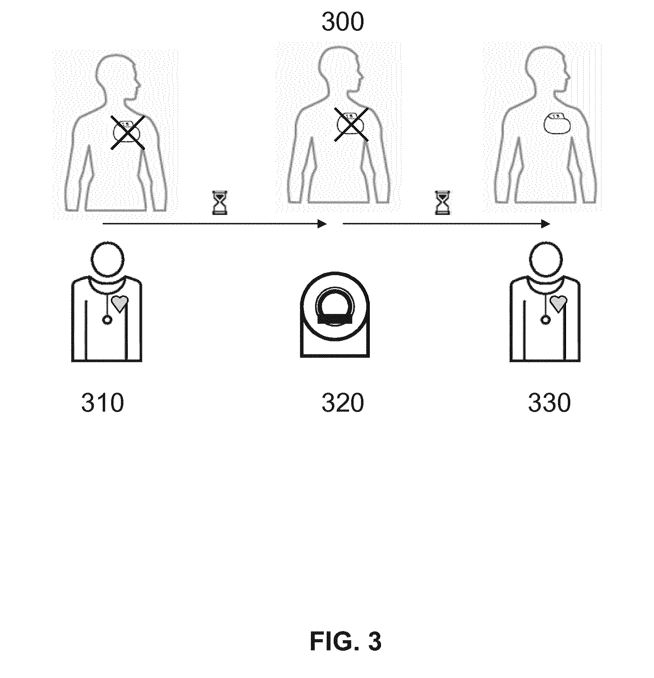

In Figur 3 ist ein typischer Ablauf einer MRT-Untersuchung eines ICD-Patienten ohne erfindungsgemäßes Gerät dargestellt. Ein ICD-Patient 300 wird vor der geplanten MRT-Untersuchung von einem Kardiologen 110 nachgesorgt und der ICD ausgeschaltet. Mit einer zeitlichen Verzögerung von Stunden bis wenigen Tagen erfolgt die MRT-Untersuchung bei einem Radiologen 120. Nach einer weiteren Verzögerung wird der Patient wieder vom Kardiologen 130 betreut und der ICD wieder eingeschaltet. Während der gesamten Zeit beginnend mit der MRT-Untersuchung durch einen Radiologen 120 bis hin zum Wiedereinschalten des ICD durch den Kardiologen 130 ist der Patient ohne den Schutz des implantierten Defibrillators und weitgehend ohne Rhythmusmonitoring. Derzeit wird dieses verbleibende Restrisiko, gemessen an dem Nutzen der MRT-Untersuchung in Kauf genommen.

Durch den Einsatz eines MRT-Sensors 82 kann der dargestellte Ablauf zwar derart verändert werden, dass nun die MRT-Einstellung nur unmittelbar während der MRT-Untersuchung wirksam wird und die abschließende Nachsorge durch den Kardiologen 130 zur Rückprogrammierung entfallen kann. Allerdings kann der Zeitpunkt der ersten Nachsorge durch den Kardiologen 110 zur Festlegung des MRT-Programmes nicht beliebig lange vor der eigentlichen MRT-Untersuchung durch den Radiologen 120 stattfinden, da sich die Konditionen des Patienten ändern und damit auch eine Anpassung des MRT-Programmes notwendig wird.

Als Beispiel sei hier die Notwendigkeit einer antibradykarden Stimulation genannt. Patienten, die keine Stimulation benötigen, sollten grundsätzlich im AUS-Mode im MRT untersucht werden, um so die Gefahr einer Arrhythmieinduktion zu vermeiden. Patienten, die eine Stimulation benötigen, müssen jedoch in einer asynchronen Betriebsart (VOO, DOO) im MRT stimuliert werden. Die jeweilige Schrittmacherabhängigkeit kann sich jedoch im Laufe der Zeit verändern.

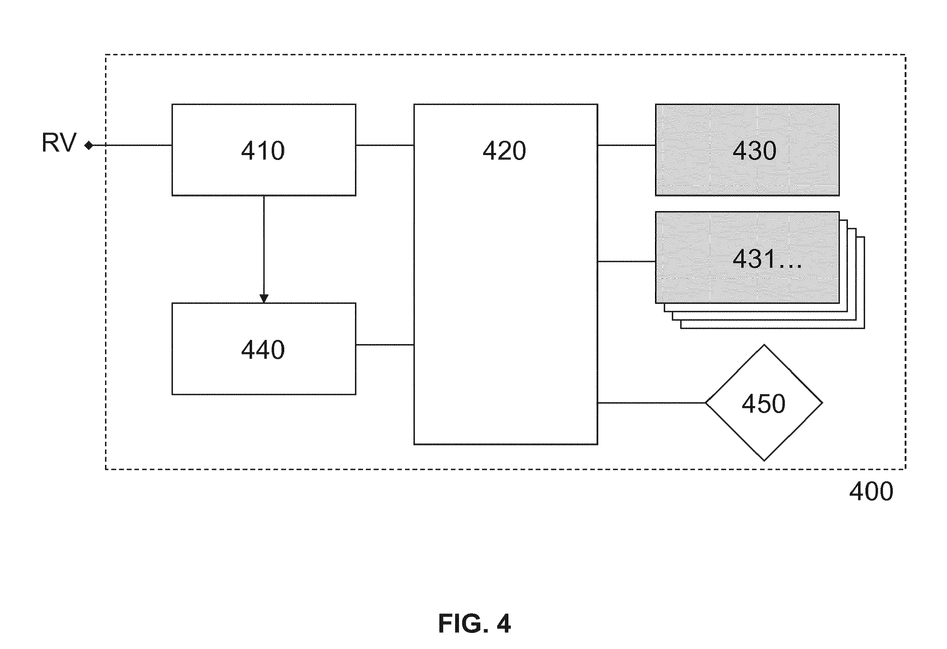

In Figur 4 ist ein gegenüber Figur 1 vereinfachtes mögliches Blockschaltbild eines solchen elektronischen implantierbaren medizinischen Gerätes 400 dargestellt. Das implantierbare medizinische Gerät 400 ist hier z.B. mit einer rechtsventrikulären Elektrodenleitung (RV) verbunden. Diese wiederum ist mit einer Wahrnehmungs- und Stimulationseinheit 410 zur antibradykarden Stimulation verbunden. Eine erfindungsgemäße Steuereinheit 420 stellt zunächst im Normalbetrieb das hinterlegte Programm zur bedarfsgesteuerten VVI-Stimulation ein. Zusätzlich werden in einer Statistikeinheit 440 Stimulations- und Wahrnehmungsstatistiken in einem Ringspeicher z.B. für die zurückliegenden 7 Tage aufgezeichnet (Anteil Stimulation oder Ergebnisse eines zyklischen Eigenrhythmustests, ...).

Für den Fall einer MRT-Untersuchung kann der nachsorgende Anwender erfindungsgemäß nun mindestens zwei Programmspeicher 430 und 431 mit einer für die MRT-Untersuchung prinzipiell geeigneten Programmierung belegen. So kann z. B. im ersten Programmspeicher 430 ein Steuerprogramm ohne Stimulation (AUS-Mode) und im zweiten Programmspeicher 431 ein Steuerprogramm mit einer asynchronen Ventrikelstimulation (VOO-Mode) hinterlegt werden. Die Programmspeicher 430 und 431 können Speicherbereiche in der Speichereinheit 80 sein, die entsprechende Steuerparameter oder Steuerprogramme und ggf. diesen zugeordnete Auswahlkriterien enthalten.

Erkennt das Implantat nun eine MRT-Umgebung mittels des integrierten MRT-Sensors 450, wertet die Steuereinheit 420 zunächst die Daten der Statistikeinheit 440 aus und wählt auf dieser Basis das für die Untersuchung am besten geeignete Programm aus (430, 431, ...). So könnte z.B. der AUS-Modus aus Steuerprogramm 1 im Programmspeicher 430 gewählt werden, wenn die Statistikdaten keinen Stimulationsbedarf des Patienten erkennen lassen und andernfalls das Steuerprogramm 2 im Programmspeicher 431 mit dem VOO-Modus.

Die Erfindung ermöglicht es dem Anwender, einmalig mehrere MRT-Programme für den jeweiligen Patienten zu hinterlegen, so dass die Notwendigkeit einer relativ zeitnahen Nachsorge des Implantates vor der MRT-Untersuchung entfällt.

## Patentansprüche

1. MRT taugliches elektronisches implantierbares Gerät mit einer Steuereinheit (54, 420) und einer Speichereinheit (80), in der Programminformation zu die Funktion der Steuereinheit (54, 420) steuernden Steuerprogrammen und/oder Steuerparameter, sowie wenigstens zeitweise jeweils aktuelle Zustandsparameter gespeichert sind, sowie mit einem MRT-Sensor (82), der mit der Steuereinheit (54, 420) verbunden und ausgebildet ist, auf eine Positionierung des implantierbaren elektronischen Gerätes innerhalb oder in unmittelbarer Umgebung eines MRT-Gerätes anzusprechen und diese anzuzeigen,
**dadurch gekennzeichnet, dass** das elektronische implantierbare Gerät (100; 400) eine Statistikeinheit (440) aufweist, die mit der Steuereinheit (54, 420) verbunden oder Teil derselben ist und die ausgebildet ist, jeweils vor einem jeweiligen Ansprechen des MRT-Sensors (82) vorliegende aktuelle Zustandsparameter zu erfassen, wobei die Steuereinheit (54, 420) ausgebildet ist, bei Ansprechen des MRT-Sensors (82) mittels Auswertung der von der Statistikeinheit (440) erfassten Zustandsparameter ein durch diese Zustandsparameter indiziertes Steuerprogramm und/oder durch diese Zustandsparameter indizierte Steuerparameter auszuwählen und solange beizubehalten, solange der MRT-Sensor (82) eine Positionierung des elektronischen implantierbaren Gerätes (100; 400) innerhalb oder in unmittelbarer Umgebung eines MRT-Gerätes anzeigt.

2. Elektronisches implantierbares Gerät gemäß Anspruch 1, **dadurch gekennzeichnet, dass** das elektronische implantierbare Gerät (100; 400) ein implantierbarer Pulsgenerator (IPG) und/oder ein implantierbarer Cardioverter/Defibrillator (ICD) und/oder ein Herzstimulator für eine kardiale Resynchronisationstherapie (CRT) oder ein Neurostimulator ist.

3. Elektronisches implantierbares Gerät gemäß Anspruch 1 oder 2, **dadurch gekennzeichnet, dass** das elektronische implantierbare Gerät (100; 400) ein Herzstimulator ist und ein in der Speichereinheit (80) abgelegtes und von der Steuereinheit (54, 420) wählbares Steuerprogramm so konfiguriert ist, dass dieses Steuerprogramm ein Auslösen von Stimulationsimpulsen unterbindet.

4. Elektronisches implantierbares Gerät gemäß wenigstens einem der Ansprüche 1 bis 3, **dadurch gekennzeichnet, dass** das elektronische implantierbare Gerät (100; 400) ein Herzstimulator ist und ein in der Speichereinheit (80) abgelegtes und von der Steuereinheit (54, 420) wählbares Steuerprogramm so konfiguriert ist, dass es eine asynchrone Stimulation in einem asynchronen Stimulationsmodus wie VOO oder DOO bewirkt.

5. Elektronisches implantierbares Gerät gemäß wenigstens einem der Ansprüche 1 bis 4, **dadurch gekennzeichnet, dass** das elektronische implantierbare Gerät (100; 400) ein Herzstimulator ist und die Statistikeinheit (440) einen Stimulationstrendspeicher umfasst oder mit einem solchen verbunden ist, wobei die Statistikeinheit (440) oder die Steuereinheit (54, 420) ausgebildet ist, ein nach Ansprechen des MRT-Sensors anzuwendendes Steuerprogramm oder nach Ansprechen des MRT-Sensors anzuwendende Steuerparameter unter Berücksichtigung von mehreren in dem Stimulationstrendspeicher in einem Zeitraum vor einem jeweiligen Ansprechen des MRT-Sensors (82) abgelegten Zustandsparametern auszuwählen.

6. Elektronisches implantierbares Gerät gemäß Anspruch 5, **dadurch gekennzeichnet, dass** der Stimulationstrendspeicher Zustandsparameter enthält, die die Abgabe von Stimulationsimpulsen und/oder die Frequenz der Abgabe von Stimulationsimpulsen widerspiegeln.

7. Elektronisches implantierbares Gerät gemäß wenigstens einem der Ansprüche 1 bis 6, **dadurch gekennzeichnet, dass** das elektronische implantierbare Gerät (100; 400) ein Herzstimulator ist und die Statistikeinheit (440) und/oder die Steuereinheit (54, 420) ausgebildet ist, ein nach Ansprechen des MRT-Sensors (82) anzuwendendes Steuerprogramm oder nach Ansprechen des MRT-Sensors (82) anzuwendende Steuerparameter unter Berücksichtigung eines von dem Herzstimulator in einem Zeitraum vor einem jeweiligen Ansprechen des MRT-Sensors (82) erfassten Eigenrhythmus auszuwählen.

8. Elektronisches implantierbares Gerät gemäß wenigstens einem der Ansprüche 1 bis 7, **dadurch gekennzeichnet, dass** das elektronische implantierbare Gerät (100; 400) ein Herzstimulator ist und die Statistikeinheit (440) und/oder die Steuereinheit (54, 420) ausgebildet ist, ein nach Ansprechen des MRT-Sensors (82) anzuwendendes Steuerprogramm oder nach Ansprechen des MRT-Sensors (82) anzuwendende Steuerparameter unter Berücksichtigung von von dem Herzstimulator in einem Zeitraum vor einem jeweiligen Ansprechen des MRT-Sensors (82) erfassten Extrasystolen auszuwählen.

9. Elektronisches implantierbares Gerät gemäß wenigstens einem der Ansprüche 1 bis 8, **dadurch gekennzeichnet, dass** die Speichereinheit (80) ein Auswahlprogramm enthält, dass die Auswahl eines nach Ansprechen des MRT-Sensors (82) anzuwendenden Steuerprogramms oder nach Ansprechen des MRT-Sensors (82) anzuwendender Steuerparameter durch die Statistikeinheit (440) und/oder die Steuereinheit (54, 420) steuert.

10. Elektronisches implantierbares Gerät gemäß Anspruch 9, **dadurch gekennzeichnet, dass** die Speichereinheit (80) neben den jeweiligen Steuerprogrammen und/oder Steuerparametern jeweils diesen Steuerprogrammen und/oder Steuerparametern zugeordnete Auswahlkriterien enthält, die die Funktionsweise der Steuereinheit (54, 420) oder der Statistikeinheit (440) beim Auswählen des jeweiligen Steuerprogramms oder der jeweiligen Steuerparameter bestimmen.

11. Elektronisches implantierbares Gerät gemäß wenigstens einem der Ansprüche 1 bis 10, **dadurch gekennzeichnet, dass** das implantierbare elektronische Gerät (100; 400) ausgebildet ist, zyklisch Testalgorithmen auszuführen, um Auswahlkriterien für die Auswahl eines nach Ansprechen des MRT-Sensors (82) anzuwendenden Steuerprogramms oder nach Ansprechen des MRT-Sensors anzuwendender Steuerparameter regelmäßig automatisch zu aktualisieren.

12. Elektronisches implantierbares Gerät gemäß wenigstens einem der Ansprüche 1 bis 11, **dadurch gekennzeichnet, dass** das implantierbare elektronische Gerät (100; 400) derart ausgebildet ist, dass es einen oder mehrere Steuerparameter oder ein oder mehrere Steuerprogramme in Abhängigkeit von erfassten Zustandsparametern modifiziert.

13. Elektronisches implantierbares Gerät gemäß wenigstens einem der Ansprüche 1 bis 12, **dadurch gekennzeichnet, dass** der MRT-Sensor (82) ein Magnetfeldsensor und/oder ein Gradientenfeldsensor und/oder ein Hochfrequenz-Feldsensor und/oder ein Lagesensor und/oder ein (Lorentz-)Vibrationssensor und/oder ein Sensor ist, der charakteristische Spannungsverläufe überwacht.
